# EUROPEAN PATENT APPLICATION

(11) **EP 2 221 617 A1**
(43) Date of publication of application: **25.08.2010**
(21) Application number: 09153346.3
(22) Date of filing: 20.02.2009
(51) Int. Cl.: G01N 30/86, G06F 19/00

(54) **A method, a system and a computer program for data processing for an automatic extraction of respective peaks in a chromatographic spectrum**

(71) Applicant: Nederlandse Organisatie voor Toegepast -Natuurwetenschappelijk Onderzoek TNO, 2628 VK Delft (NL)
(72) Inventor: Jellema, Renger Henk, 3532 TL Utrecht (NL); Vogels, Jacobus Thomas Wilhelmus Elisabeth, 3700 AJ Zeist (NL); van der Kloet, Frans Meindert, 3700 AJ Zeist (NL); Thissen, Uwe Maria Johannes, 3700 AJ Zeist (NL)
(74) Representative: Hatzmann, Martin

(57) **Abstract**

The invention relates to a system and a computer program for data processing for automatic extraction of respective peaks in a chromatographic spectrum. The system (80) may comprise a processor (82), a suitable database (83) for deconvoluting a chromatographic spectrum. In order to access chromatographic spectral data the system (80) comprises an input unit (81), which may be connectable to an output of a suitable measurement system (85) arranged for generating chromatographic spectra. The system (80) according to the invention further comprises a computer program product (84) for deconvoluting the chromatographic spectrum into the respective peaks based on a plurality of characteristic peaks using multivariate statistical analysis, wherein the plurality of characteristic peaks is selected from the database using prior knowledge about expected constituents of the chromatographic spectrum.

## Description

### FIELD

The invention relates to a method of data processing for automatic extraction of respective peaks in a chromatographic spectrum. The invention further relates to a system for data processing for automatic extraction of respective peaks in a chromatographic spectrum. The invention still further relates to a computer program for data processing for automatic extraction of respective peaks in a chromatographic spectrum.

### BACKGROUND

An embodiment of a method as is set forth in the opening paragraph is known from US 2005/0273276. In the known method a chromatographic spectrum comprising a plurality of convoluted peaks is processed using four dedicated software packages. First software package is arranged for an initial analysis of the chromatographic spectra using retention time analysis and mass spectra analysis. As a first output deconvoluted peaks are generated. The second software package is arranged to receive results of the first software package for generating a second result further identifying and/or confirming the identity of the target compounds of the first output. The third software relates to the NIST02 library software package and is arranged to receive the second result for generating a third result still further confirming the identity of the target compounds. Finally, the fourth software package is arranged to receive the first, the second and the third results for confirming the identity of the target compound by sorting the first, second and the third results according to predefined criteria. For example, retention time, four ion analysis and chemical abstracts service number may be used for such predefined criteria.

It is a disadvantage of the known method that a complex, multi-step identification procedure has to be followed for deconvoluting and identifying individual spectra in the total ion chromatogram.

### SUMMARY OF THE INVENTION

It is an object of the invention to provide a method for an automatic deconvolution of individual peaks in the chromatogram, which is simple, yet robust and reliable.

To this end the method of data processing for an automatic extraction of respective peaks in a chromatographic spectrum according to the invention comprises the steps of:
- accessing a database of characteristic peaks;
- deconvoluting the chromatographic spectrum into the respective peaks based on a plurality of characteristic peaks using multivariate statistical analysis, wherein the plurality of characteristic peaks is selected from the said database using prior knowledge about expected constituents of the chromatographic spectrum.

It is found that using prior knowledge, for example knowledge on expected constituents in the chromatographic spectrum under consideration, substantial computation time may be saved without compromising deconvolution accuracy. In particular, according to the method of the invention, the characteristic peaks are used for fitting the integral chromatographic spectrum using multivariate statistical analysis. It will be appreciated that methods of the multivariate statistical analysis are known per se and application thereof to chromatographic spectra falls within ordinary skill of the person skilled in the art.

It is found to be advantageous to fit the chromatographic spectra using multivariate statistical analysis as in course thereof respective characteristic spectra or portions thereof may be suitably shifted along a fitting axis, for example, along retention time axis. As a result, the method according to the invention may be more flexible and, thus, more successful in deconvoluting distorted spectra, as no perfect match between the characteristic spectra from the database and a best fit for the chromatographic spectrum may be required. The method known from US 2005/0273276 may provide inferior results, as it operates using some mathematical processes resolving unresolved compounds based on standard library data, wherein characteristic peaks may be treated as rigid entities having pre-determined and non alterable positions along a fitting axis.

In an embodiment of the method according to the invention, the method comprises the step of assigning a confidence level to the extracted peaks in the chromatogram.

It is found that for some chromatographic spectra particular peaks may not be fully or adequately resolved. For example, when the sample to be analyzed comprises unexpected peaks, or when the background level is too high for some areas. It is, therefore, found to be particularly advantageous to provide the method according to the invention with an internal quality check, by allowing an automatic assignment of a confidentiality level to the extracted peaks. In this way, when a peak with a confidentiality level below a pre-determined threshold is signalled, an automatic or manual re-fit may be carried out.

Preferably, the method according to the invention generates an automatic report based on the result of deconvolution of the chromatographic spectrum. It is found to be advantageous to supplement peaks identified in the report with their respective confidence level. However, it may be also possible that only peaks with an unacceptable confidence level are marked, for example peaks having a confidence level below a pre-determined level may be underlined, labelled or highlighted in any other suitable way.

In a further embodiment of the method according to the invention, wherein the chromatogram comprises a residual signal post extraction of the respective peaks, the method according to the invention further comprises the step of extracting residual peaks from the chromatogram based on a residual signal.

It is found to be advantageous for extraction of peaks corresponding to trace elements to first deconvolute major peaks including their intrinsic sub-peaks, followed by deconvolution of residual peaks. In this way accuracy for identification of residual peaks may be increased.

A computer program product according to the invention comprises instructions for causing a processor to carry out the steps of the method as is set forth with reference to the foregoing.

A system according to the invention comprises:
- input for accessing a database of characteristic peaks;
- processor for deconvoluting the chromatographic spectrum into the respective peaks based on a plurality of characteristic peaks using multivariate statistical analysis, wherein the plurality of characteristic peaks is selected from the said database using prior knowledge about expected constituents of the chromatographic spectrum.

It will be appreciated that the system according to the invention may be operable at a different location with respect to a physical location of the databases of characteristic peaks. For example, the database may be provided on a specific server which may be accessible by different users, like different laboratories, hospitals or the like. Preferably, the system according to the invention comprises the database which may lead to an increased efficiency of the system as data transport is carried out locally. More preferably, the system according to the invention comprises a chromatography unit. In this case data analysis may be performed substantially instantly after generating the chromatographic spectra, which may be advantageous, especially when it is desired to repeat the step of chromatography on the same or on a similar sample for improving deconvolution accuracy.

These and other aspects of the invention will be discussed in more detail with reference to drawings, wherein like reference numerals refer to like elements. It will be appreciated that the drawings are presents for illustrative purposes and may not be used for limiting the scope of the appended claims.

### BRIEF DESCRIPTION OF THE DRAWINGS

Figure 1 depicts in a schematic way an embodiment of a chromatographic spectrum comprising three convoluted peaks;
Figure 2 depicts in a schematic way an embodiment of resolved peaks substantially constituting the chromatogram of Figure 1;
Figure 3 depicts in a schematic way convoluted spectra for peaks 2 and 3 of Figure 1;
Figure 4 depicts in a schematic way characteristic peaks for peak 1 of Figure 1;
Figure 5 depicts in a schematic way characteristic peaks for peak 2 of Figure 1;
Figure 6 depicts in a schematic way characteristic peaks for peak 3 of Figure 1;
Figure 7 depicts in a schematic way an embodiment of a flow-chart of a computer program product according to the invention;
Figure 8 depicts in a schematic way an embodiment of a system according to the invention.

### DETAILED DESCRIPTION OF THE DRAWINGS

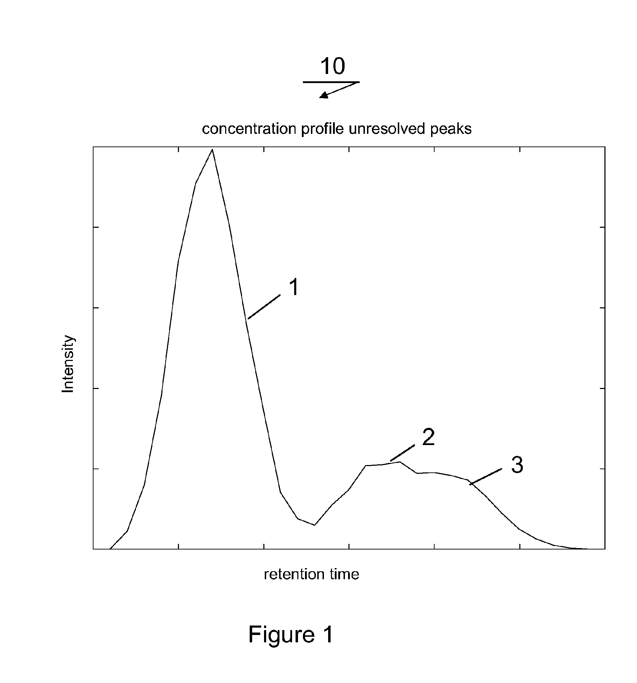

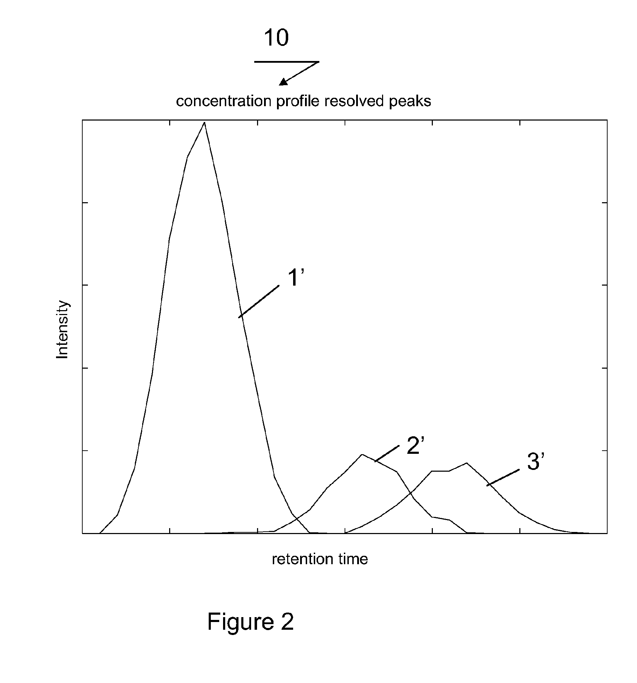

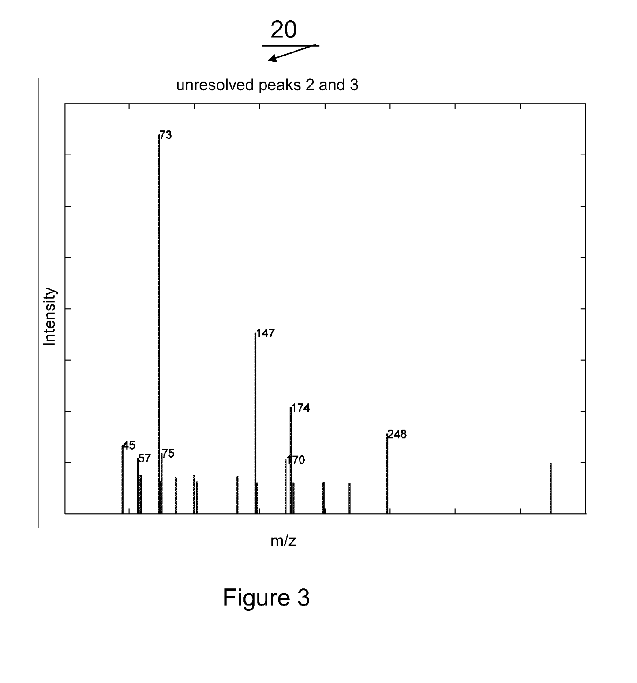

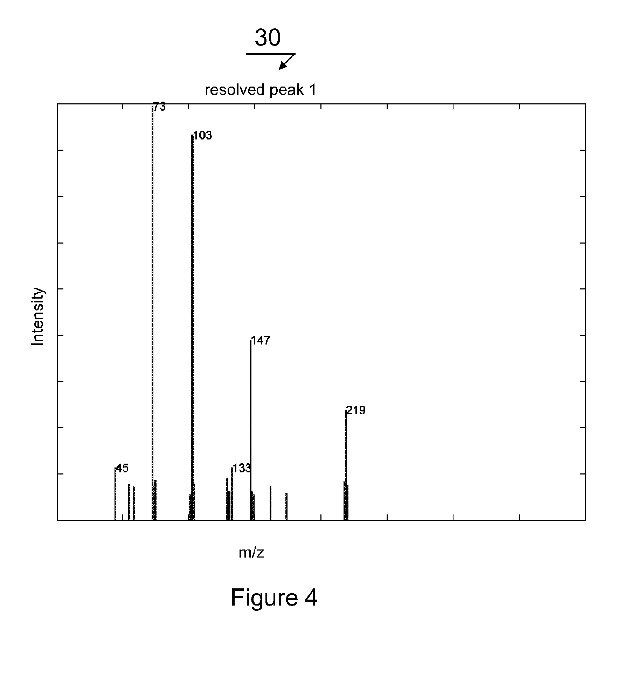

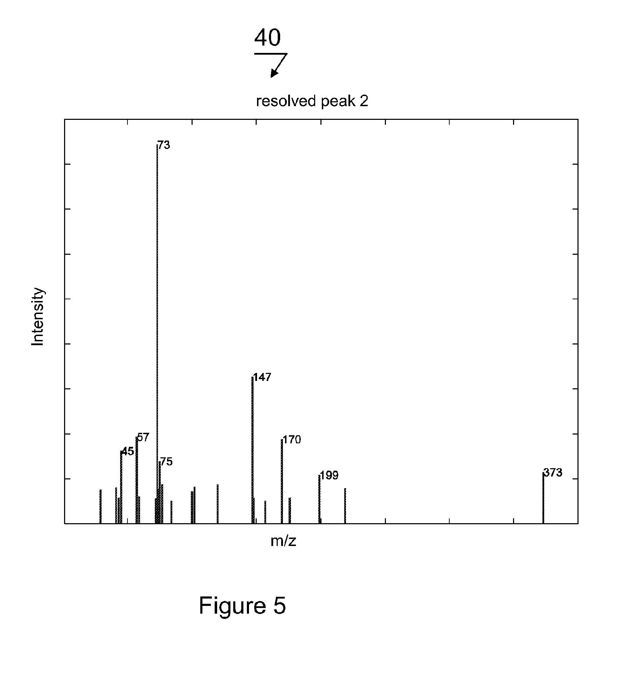

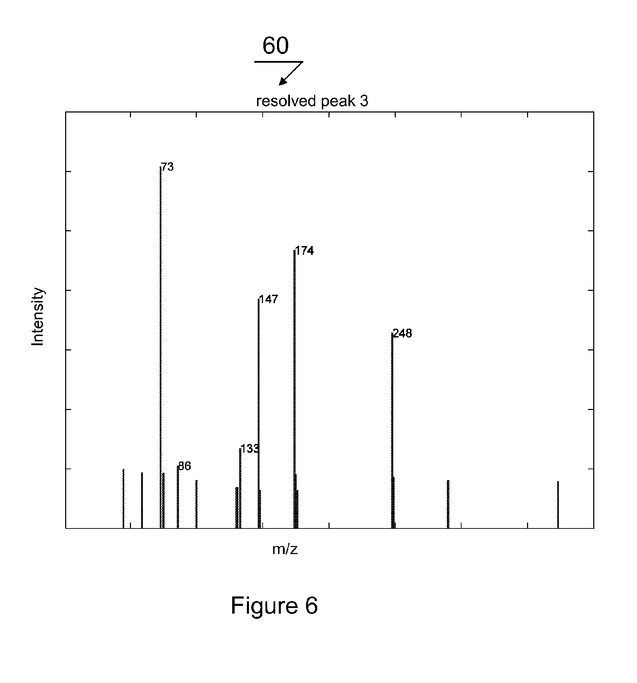

Figure 1 depicts in a schematic way an embodiment of a chromatogram 10 comprising three convoluted peaks 1, 2 and 3, which are presented as a function of retention time. The chromatogram 10 may be generated by means of gas chromatography which generally involves a vaporised sample which is injected onto a head portion of a chromatographic column. The sample is usually transported through the column by a flow on inert, gaseous mobile phase. The column may comprise a liquid stationary phase which is adsorbed onto a surface of an inert solid. The detector used for generating chromatographic data may refer to a so-called concentration-dependent detector, which is known per se. It will be appreciated that the methodology of the gas chromatography is known per se. It will be further appreciated that the chromatogram 10 may comprise more or less individual peaks, wherein a degree of mutual overlap between such peaks may also be different from sample to sample. Figure 2 depicts in a schematic way an embodiment of resolved peaks 1', 2', 3' substantially constituting the chromatogram 10 of Figure 1. Figure 3 depicts in a schematic way convoluted spectra 20 for peaks 2 and 3 of Figure 1, wherein individual peaks constituting the convoluted spectra may be taken from a suitable database of characteristic peaks. Figure 4 depicts in a schematic way characteristic peaks 30 constituting the spectrum of peak 1 of Figure 1 and Figure 5 depicts in a schematic way characteristic peaks 40 constituting the spectrum of peak 2 of Figure 1. Figure 6 depicts in a schematic way characteristic peaks 60 constituting the spectrum of peak 3 of Figure 1. It will be appreciated that the characteristic peaks 30, 40 and 60 in accordance with the invention are taken from the a-priori compiled database of characteristic peaks which are most probably present in the sample under consideration. It is possible that such database is compiled in an empirical way, i.e. based on experience of a particular lab, or an experience with particular samples. It is also possible that the database is compiled using standard databases of chromatograms of chemical species, or based on an abstract of such standard database.

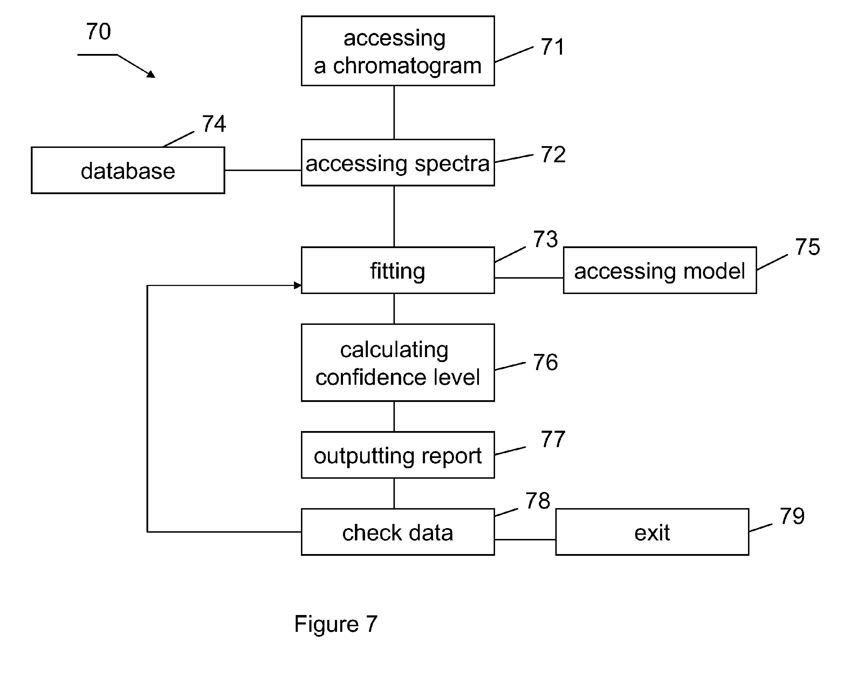

Figure 7 depicts in a schematic way an embodiment of a flow-chart of a computer program product according to the invention. The computer program product 70 may relate to an executable file comprising instructions for causing a processor of a suitable computer to carry out the steps of the method according to the invention. Accordingly, at step 71 the computer program product, embodiment of which is discussed herein below with reference to a flow-chart representation, may comprise an instruction for causing the processor to access a suitable chromatographic spectrum. Such operation may be carried out by setting suitable input means into a data 'receive' mode. For example, a suitable chromatography spectrum may be accessed from a memory of a computer, or, alternatively from a remote site. For the latter local area network or internet facilities may be used.

After the chromatographic spectra data are accessed, the computer program according to the invention by means of instruction 72 causes the processor to access characteristic spectra from a database 74, wherein the characteristic spectra are supposed to be used for deconvoluting the chromatographic spectrum. In order to purposefully select the characteristic spectra, the computer program may access a data identifier (not shown), which may be stored in the file of the chromatographic spectrum for source identification purpose. For example, should the chromatographic spectrum correspond to a medical sample, the corresponding data may be stored in a pre-determined format, wherein identification of expected sample constituents are notified.

At step 73, the computer program comprises an instruction for the processor for carrying out a step of numerical fitting of the chromatographic spectrum for deconvoluting peaks. For this purpose at step 75 a suitable fitting model, of a multivariate statistical analysis, like equations of a regression analysis, may be accessed. After this stem peaks constituting the chromatographic spectrum accessed at step 71 are deconvoluted.

In order to quantify accuracy of the deconvolution, the computer program according to the invention comprises instruction 76 for causing the processor to calculate a confidence level related to accuracy of deconvolution, for example related to accuracy of identification of a sub-peak.

At step 77 the computer program according to the invention may cause the processor to generate a report in a suitable form, for example, the report may relate to a table, a hard-copy print, or a file stored in a suitable format, for example in a HTML format. After the report is generated, the computer program may proceed to a further instruction 78 for checking output data, wherein peaks having confidence level lower than a pre-set threshold confidence level are suitably marked. Although in a preferred embodiment the computer program could proceed automatically to refitting at least the peaks with inferior confidence level, it is also possible that such peaks may be refitted manually. Finally, the computer program stops its operation by proceeding to the exit instruction.

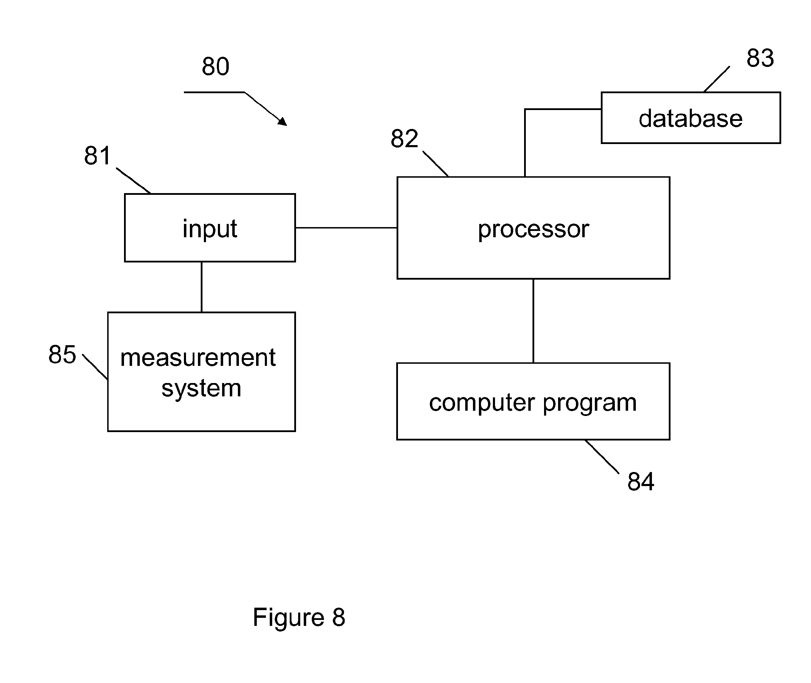

Figure 8 depicts in a schematic way an embodiment of a system according to the invention. A system 80 according to the invention comprises a processor 82, which, preferably, makes part of a suitable computer. The system 80 may further comprise a suitable database 83 of the characteristic peaks which may be assessed automatically by the system upon deconvoluting a chromatographic spectrum. The database may be stored in a memory unit, or may be provided on a suitable data carrier, like a CD-ROM, a flash memory disk or any other suitably readable medium. It is further possible that the database is stored remotely with respect to the processor and that processor establishes a data communication link with the database when a chromatographic spectrum is to be resolved.

In order to access data the system 80 comprises an input unit 81, which may be connectable to an output (not shown) of a suitable measurement system 82 arranged for generating chromatographic spectra. Preferably, such measurement system forms part of the system 80 according to the invention.

The system 80 according to the invention further comprises a computer program product 84, which is explained in detail with reference to Figure 7, said computer program product 84 being arranged to operate a processor 82 in accordance with the method of the invention. The system 80 may further comprise suitable display for providing a feed-back to a user, as well as other suitable periphery units, like printers, modems, or the like.

It will be appreciated that while specific embodiments of the invention have been described above, that the invention may be practiced otherwise than as described. In addition, isolated features discussed with reference to different figures may be combined.

## Claims

1. Method of data processing for an automatic extraction of respective peaks in a chromatographic spectrum, comprising the steps of:
- accessing a database of characteristic peaks;
- deconvoluting the chromatographic spectrum into the respective peaks based on a plurality of characteristic peaks using multivariate statistical analysis, wherein the plurality of characteristic peaks is selected from the said database using prior knowledge about expected constituents of the chromatographic spectrum.

2. Method according to claim 1, comprising the step of assigning a confidence level to the extracted peaks in the chromatogram.

3. Method according to claim 2, wherein in the confidence level a threshold level is set.

4. Method according to claim 2 or 3, further comprising a step of generating a report on the respective peaks, wherein at least the peaks with a confidence level below the threshold level are marked.

5. Method according to any one of the preceding claims, wherein the chromatographic spectrum comprises a residual signal post extraction of the respective peaks, the method further comprising the step of extracting residual peaks from the chromatographic spectrum based on a residual signal.

6. Method according to any one of the preceding claim, wherein the multivariate statistical analysis comprises a step of relative shifting of the respective peaks.

7. Method according to any one of the preceding claims, wherein the chromatographic spectrum is provided in an electronic file comprising data at least partially identifying expected constituents of the chromatographic spectrum, the method further comprising the step of using said data for the prior knowledge.

8. Computer program product comprising instructions for causing a processor to carry out the steps of the method as claimed in claims 1 - 7.

9. System for data processing for an automatic extraction of respective peaks in a chromatographic spectrum, comprising:
- input for accessing a database of characteristic peaks;
- processor for deconvoluting the chromatographic spectrum into the respective peaks based on a plurality of characteristic peaks using multivariate statistical analysis, wherein the plurality of characteristic peaks is selected from the said database using prior knowledge about expected constituents of the chromatographic spectrum.

10. System according to claim 9, further comprising the database of characteristic peaks.

11. System according to claim 9 or 10 further comprising a gas chromatography unit.
